# EUROPEAN PATENT SPECIFICATION

(11) **EP 2 752 665 B1**
(45) Date of publication and mention of the grant of the patent: **07.12.2016**
(21) Application number: 14150052.0
(22) Date of filing: 02.01.2014
(51) Int. Cl.: C12N 9/20, G01N 33/574

(54) **Novel biomarker for diagnosis of lung cancer**
Neuartiger Biomarker zur Diagnose von Lungenkrebs
Nouveau biomarqueur pour le diagnostic du cancer du poumon

(30) Priority: 04.01.2013 KR 20130001267
(43) Date of publication of application: 09.07.2014
(73) Proprietor: Seoul National University R&DB Foundation, Seoul 151-742 (KR)
(72) Inventor: Cho, Je Yoel, 135-506 Seoul (KR)
(74) Representative: Peter, Julian

(56) References cited:
- WO-A2-2011/150994
- US-A1- 2006 094 048

## Description

### [BACKGROUND OF THE INVENTION]

### 1. Field of the Invention

The present invention relates to a novel biomarker for diagnosis of lung cancer, and more particularly, to a composition for diagnosis of lung cancer, including GPLD1 protein with specifically decreased expression in a lung cancer patient and a diagnosis method for lung cancer using the same.

### 2. Description of the Related Art

As can be known from the statistics reporting that one among four persons in the population of Korea has died due to cancer, cancer has come to the fore as the most serious threat factor to national health, in recent years. Owing to various environmental changes including an aging population, it is expected that cancer-related health threats and death will be further increased. In particular, according to the 2009 data from the national statistics office in relation to the mortality of types of cancer for the past 10 years in Korea, lung cancer showed the highest increase.

Lung cancer is one among cancers with high lethality all over the world as well as domestically, and such a trend is caused by less subjective symptoms and an absence in early diagnosis methods having high sensitivity. Currently, lung cancer has a relatively high diagnosis dependence upon imaging methods (i.e., X-ray, CT, MRI, etc.) and there have been discovered a few precedents for materials which may be potentially used as a biochemical indicator.

The term 'biomarker' refers to all of physical and/or biochemical indicators capable of making a diagnosis of physiological and/or pathological conditions of a human body. At present, a paradigm in cancer treatment is changing from developing anti-cancer drugs for direct treatment of cancer toward inducing early discovery thereof and thus enabling effective treatment and continuous monitoring. Therefore, advanced early diagnosis and monitoring methods are much needed for the foregoing change, and an appropriate biomarker may be the most necessary part in such an advanced early diagnosis method as described above Existing methods for the development of new medicaments involve a process wherein capital of at least 800 million dollars is invested while requiring at least 10 years from the screening of chemical substances, detection and determination of candidate materials, a pre-clinical stage, three following clinical study stages, and up to commercialization. In contrast, commercialization of a diagnostic kit using a biomarker can lead to commercialization of an anti-cancer agent with less capital investment than the above in a short period of time.

For lung cancer, although a number of articles regarding candidate materials for potential biomarkers have been published and new biomarker candidate proteins are continuously under research and development, there is still no precedent for commercialization of such proteins as a biomarker specific to the diagnosis of lung cancer.

Meanwhile, glycosylphosphatidylinositol (GPI) specific phospholipase D1 (GPLD1) is an enzyme present in a large amount in blood plasma obtained from mammals, which is known to cleave GPI-anchored protein positioned at surfaces of cells and allow the cleaved proteins to have physiological activity with respect to each cell. Currently, crypto-1 and prostatin only have been discovered as proteins targeted by GPLD1 and are merely known to participate in lipid metabolism, although those are present in a great amount in the blood plasma. Consequently, definite functions of the foregoing proteins have been little investigated and no report has been disclosed specifically regarding lung cancer.
WO 2011/150994 A2 discloses a serum and tissue biomarkers of human hepatocellular carcinoma (HCC) as well as GPLD1 antibodies.
US2006/094048 A discloses a system, method and product for multiple wavelength detection using single source excitation.

### [SUMMARY OF THE INVENTION]

Accordingly, it is an object of the present invention to provide a novel marker for diagnosis of lung cancer including glycosylphosphatidylinositol (GPI) specific phospholipase D1 (GPLD1) protein.

In order to accomplish the above object, the present invention provides the use of a composition according to claim 1.

Further, the present invention uses a composition for diagnosis (hereinafter, often referred to as a 'diagnostic composition') of lung cancer, including a primer or probe specific to a nucleic acid encoding GPLD1 protein.

According to the present invention, lung cancer is squamous epithelium cell lung cancer (SQLC).

The present invention also uses a kit for diagnosis of lung cancer, a micro-array, a protein chip and a DNA chip, containing the foregoing composition as an active ingredient.

The marker for diagnosis of lung cancer according to the present invention may be effectively used in early diagnosis of lung cancer, and is very useful for evaluating progression of a disease and prognosis before and after treatment of the same.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 shows results of removing albumin and IgG from each of blood serums obtained from normal persons (HEC) and squamous epithelium cell lung cancer (SQLC) patients, respectively;
FIG. 2 is graphs illustrating expression amounts of GPLD1 in liver cells (HepG2), a normal lung cell-line (L132) and lung cancer cell-lines (A549, H157, H1229, H460, and H358);
FIG. 3 shows results of Western-blot assay to identify expression of GPLD1 in each of blood serums obtained from normal persons (HEC) and squamous epithelium cell lung cancer (SQLC) patients, respectively;
FIG. 4 shows ELISA results to identify expression of GPLD1 in normal persons (HEC) and lung cancer groups (SQLC, ADC, and SCLC), respectively;
FIG. 5 shows results of Western-blot assay to exhibit expression patterns of GPLD1 in lung cancer tissue (LC) and adjacent normal tissue (N) obtained from a lung cancer patient, respectively (except for No. 866 and No. 882);
FIG. 6 shows results of densitometry analysis for quantitatively exhibiting expression patterns of GPLD1 in lung cancer tissue and adjacent normal tissue obtained from a lung cancer patient; and
FIG. 7 illustrates (A) a photograph of the lung of a normal mouse, (B) a photograph of the lung of a mouse with induced lung cancer, and (C) qualified results of expression patterns of GPLD1 in a normal group and lung cancer group through Western blot, respectively, after IV injecting lung cancer cells into a normal mouse then excising the lung (N: normal, IV: injection of lung cancer cells).

### [DETAILED DESCRIPTION OF EMBODIMENTS]

Hereinafter, the present invention will be described in detail with reference to the accompanying drawings.

The present invention provides a diagnostic composition for lung cancer, including an antibody or polypeptide specifically bound to a glycosylphosphatidylinositol (GPI) specific phospholipase D1 (GPLD1) protein having decreased expression in a blood serum or lung cancer obtained from a lung cancer patient.

The term "diagnosis" used herein means identifying the presence or characteristics of a pathological condition. With regard to the purposes of the present invention, the diagnosis may refer to identifying the onset of lung cancer.

The term "lung cancer" used herein means a malignant tumor occurring in the lung and, in histological aspects, may include all of small cell lung cancer, lung adenocarcinoma, squamous epithelium lung cancer and large cell lung cancer.

The term "cancer diagnostic marker" used herein means a material that can distinguish a cancer cell from normal cells to deliver the same, and may refer to a bio-molecule that increases or decreases in a cell under attack from cancer, as compared to normal cells. Such bio-molecules may include polypeptide, protein, nucleic acid, lipid, glycolipid, glycoprotein, glucose, or the like, but not be limited thereto. According to the present invention, the cancer diagnostic marker may include a protein with specifically decreased expression in the blood serum or tissue obtained from a lung cancer patient or a nucleic acid encoding the same. The lung cancer diagnostic marker according to the present invention may be used for diagnosis in order to distinguish patients having lung cancer from persons without the same.

The antibody used in the present invention may include a whole type antibody (hereinafter, 'whole antibody') or a functional fragment thereof. The whole antibody may be in a monomer form or a multimer form containing at least two whole antibodies bonded together. The functional fragment of the antibody may comprise an antibody having a heavy chain or light chain variable region of the whole antibody, which substantially recognizes the same epitope as that recognized by the whole antibody.

The functional fragment of the antibody described above may include, but is not limited to, single chain variable region fragments such as (scFv), (scFv)2, Fab, Fab' and F(ab')2, etc. Such a single chain variable region (scFv) refers to an antibody fragment wherein a heavy chain variable region is linked to a light chain variable region through a linker peptide to form a single chain polypeptide.

The antibody may be bound to different molecules such as enzymes, fluorescent materials, radioactive materials and/or proteins, thus being modified. The modified antibody may also be formed by chemically modifying an antibody. The above modification methods are generally used in the art. In addition, the above antibody may comprise a chimeric antibody formed by combining a non-human antibody-derived variable region with a human antibody-derived constant region or, otherwise, a humanized antibody formed by combining a human antibody-derived framework region (FR) that contains a complementarity determining region derived, from a non-human antibody, with a constant region derived from a human antibody. The antibodies described above may be prepared by any conventional method well known in the art.

The diagnostic composition of the present invention may further include any reagent known in the art and generally used for immunological assay in addition to an antibody specific to a protein. The immunological assay used herein may include any method capable of measuring antigen-antibody binding.

The present invention also provides a kit for diagnosis of lung cancer, including the above composition as an active ingredient.

The diagnostic kit may include, for example, a lateral flow assay kit based on immune-chromatography in order to detect a specific protein in a sample. The lateral flow assay kit may include : a sample pad receiving a sample thereon; a releasing pad coated with a detecting antibody; a developing membrane (for example nitrocellulose) or strip wherein the sample moves, is isolated, and undergoes antigen-antibody reaction; and an absorption pad.

The present invention also provides a micro-array for diagnosis of lung cancer, including the above composition as an active ingredient.

The micro-array is generally used to attach an antibody on the surface of a slide glass treated with a specific reagent, in order to detect a protein specifically adhered to the antibody by an antigen-antibody reaction.

Alternatively, the present invention provides a diagnostic composition for lung cancer, which includes a primer or probe specific to a nucleic acid encoding GPI specific phospholipase D1 (GPLD1) protein.

Detection of a specific nucleic acid using the primer described above may be performed by amplifying a sequence of a target gene using an amplification method such as PCR and identifying such amplification of the gene according to a conventional method known in the art. Also, detection of a specific nucleic acid using the probe described above may be performed by contacting the probe with a sample nucleic acid under desired conditions and identifying the existence of hybridized nucleic acid.

Herein, the "primer" refers to a nucleic acid sequence having a short free hydroxyl group and substantially means a short nucleic acid sequence capable of forming a base pair with a complementary template and functioning as a starting point for copying a template strand.

The "probe" used herein refers to a nucleic acid fragment such as RNA or DNA, consisting of several to several hundred bases that can be specifically bound to mRNA, and may be labeled to identify the existence of a specific mRNA. The probe may be prepared in the form of an oligonucleotide probe, single chain DNA probe, double chain DNA probe, or RNA probe, etc., and be labeled by biotin, FITC, rhodamine, DIG, etc. or otherwise, by radioactive isotopes.

In addition, the probe may be labeled by a detectable material, for example, a radioactive label providing a desired signal and having a sufficient half-life. The labeled probe may be hybridized to a nucleic acid on a solid support, as is known in the art.

A method of detecting a specific nucleic acid using the probe or primer described above may include, but is not limited to, polymerase chain reaction (PCR), DNA sequencing, RT-PCR, primer extension, oligonucleotide extension assay, allele specific PCR, RNase mismatch cleavage, single strand conformation polymorphism, SCCP and heteroduplex simultaneous assay, denaturing gradient gel electrophoresis (DGGE), denaturing high-pressure liquid chromatography (MHPLC), hybridization, DNA chip, or the like. Examples of the hybridization may include northern hybridization, in-situ hybridization, a micro-array method, or the like.

The diagnostic composition for lung cancer of the present invention may further include a reagent generally used for detection of the nucleic acid described above. For instance, deoxynucleotide triphosphate (dNTP), a heat-resistant polymerase or a metal ion salt such as magnesium chloride, which are required for PCR, may be included. Alternatively, dNTP or sequenase required for sequencing may be included.

Preferably, the diagnostic composition for lung cancer of the present invention may be provided in the form of a diagnostic kit or micro-array. For instance, a RT-PCR kit including each primer pair specific to genes encoding GPLD1 protein of the present invention, a DNA chip including a substrate that has cDNA of a marker gene or oligonucleotide attached thereto according to the present invention, and the like, may be provided but is not be limited thereto.

Hereinafter, preferred embodiments are provided to more clearly understand the present invention.

However, the following examples are only given for more concretely describing the present invention and those skilled in the art will obviously understand that a variety of modifications and alterations may be possible within the scope and technical spirit of the present invention, and such modifications and alterations are duly included in the scope of the appended claims.

### EXAMPLE

### Example 1: Serum proteomics assay

### (1-1) Serum collection

For serum proteomics assay, blood of 10 normal persons (HEC) and 10 patients with squamous epithelium cell lung cancer (SQLC) were subjected to pooling, respectively.

With regard to studies of marker proteins which are cancer-specifically expressed and generated by a material such as cytokine or environments adjacent to a cancer, some proteins such as albumin or IgG contained in a great amount in the blood must be removed beforehand. Therefore, using a protein depletion kit (ProteoPrep Immunoaffinity Albumin & IgG Depletion Kit, Sigma, USA), albumin and IgG among proteins in the serum were depleted from 20 serum samples obtained after pooling.

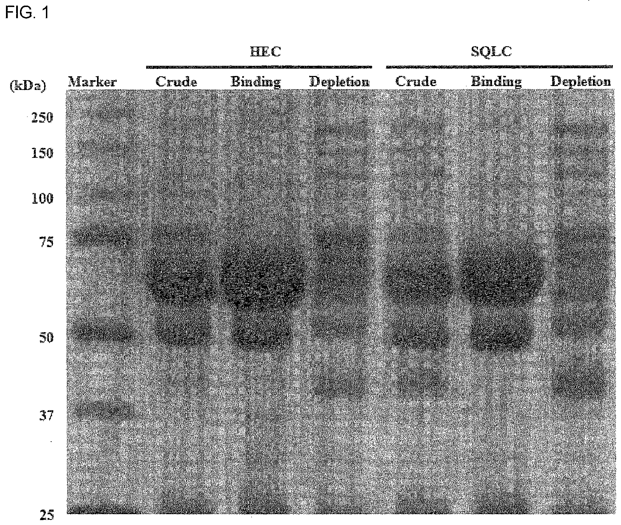

More particularly, when each serum specimen obtained as described above was loaded into a column of the kit, only albumin and IgG were combined with a resin in the column while other proteins passed through the column. Therefore, a serum sample free from the albumin and IgG was obtained by collecting the sample having passed through the column. Results of this process are shown in FIG. 1.

### (1-2) Coomassie blue staining

The foregoing obtained serum was removed by first order gel electrophoresis (1 DE-SDS-PAGE) in order to conduct an LC-MS/MS assay. After washing the gel with H₂O three times, the gel was stained using a bio-safe Coomassie staining solution (Coomassie G250 stain: Bio-Rad) while softly agitating at room temperature for 1 hour. Next, the gel was incubated in deionized distilled water for 30 minutes then washed three times with deionized distilled water for 10 minutes.

### (1-3) In-gel tryptic digestion

According to a conventional method (Heo, S.H., Lee, S.J., Ryoo, H.M., Park, J.Y., Cho, J.Y., Proteomics 2007, 7,4292-4302), in-gel tryptic digestion was executed.

After removing a protein band from a Coomassie staining gel, the remaining gel was decolorized by incubating the same in a 75 mM ammonium bicarbonate/40% ethanol (1:1) solution. Then, it was treated using a 5 mM DTT/25 mM ammonium bicarbonate at 60□ for 30 minutes for desulfurization. Next, the treated product was subjected to alkylation using 55 mM iodoacetoamide at room temperature for 30 minutes. Such treated gel pieces were dried using 100% ACN. Following this, gel pieces were rehydrated using 10 µl of a 25 mM ammonium bicarbonate buffer containing 20 µg/ml of modified sequencing grade trypsin (Roche Applied Science) then incubated at 37□ overnight. Using 0.1 % formic acid, a trypsin peptide mixture solution was eluted.

A trypsin functional peptide in the Coomassie stained gel band was analyzed with LC-MS/MS.

### (1-4) LC-ESI-MS/MS assay

Using a thermo Finnigan's ProteomeX workstation LTQ linear ion trap MS (Thermo Electron, San Jose, CA, USA) provided with NSI source (San Jose, CA), an LC-MS/MS assay was executed according to a conventional method (Heo, S.H., Lee, S.J., Ryoo, H.M., Park, J.Y., Cho, J.Y., Proteomics 2007, 7, 4292-4302).

12 ml of a peptide sample obtained after in-gel digestion was injected and loaded into a peptide trap cartridge (Agilent, Palo Alto, CA). The loaded peptide was eluted through a 10cm reverse-phase PicoFrit column filled with C18 having a pore size of 300□, and a gradient eluent was extracted. A mobile phase comprised H₂O (A) and ACN (B), wherein both of these fractions contained 0.1 %(v/v) of formic acid. A flow rate was maintained at about 200 nL/min. A concentration gradient started at 2% of B and, after 50 minutes, reached 60% of B. Thereafter, it reached 80% of B for 5 minutes, followed by 100% of A for the last 15 minutes. After driving a data-dependent acquisition (m/z: 400 to 1800) and executing a survey MS scan, an MS/MS scan was executed five times for 30 seconds while switching on a power output option. A spray voltage was 1.9 kV and a temperature of an ion migration tube was set up to 195°C. Standard impact energy was set up to 35%.

### Example 2: Semi-quantitative analysis of serum protein

Semi-quantitative proteomics is a method of using a spectral count exhibited in a process for analyzing identification of a protein after an MS/MS assay instead of using a labeling material.

According to an existing method for semi-quantitative analysis, the serum protein prepared in Example 1 was analyzed.

More particularly, after subjecting the albumin and IgG-free serum to in-gel trypsin digestion, the serum was analyzed by means of a mass analyzer (LC-ESI-MS/MS) to identify proteins in each sample. Results of comparison and analysis for respective test groups using a scaffold 2 program (Proteome Software, Inc., USA) are shown in Table 1 below. From the results, it was found that GPLD1 is specifically reduced in the squamous epithelium lung cancer. The analyzed results obtained by the mass analyzer demonstrated that 6 GPLD1 peptide hits were identified in the blood of a normal person (HEC) whereas the blood of an SQLC patient exhibited no peptide hit. Based on these results, it was demonstrated that expression of GPLD1 was decreased in the blood of the SQLC patient.

**[TABLE 1] Relative quantification of GPLD1 protein identified by LC-ESI-MS/MS assay**

| **Identified Proteins** | **IPI Number** | **Peptide Hits HEC** | **Peptide Hits SQLC** | **Healthy fold** |
|---|---|---|---|---|
| Gene_Symbol=GPLD1 Isoform 1 of Phosphatidylinositol-glycan-specific phospholipase D | IPI00299503 | 6 | 0 | - |

Furthermore, significantly increased or decreased proteins other than GPLD1 in a SQLC sample, as compared to a normal sample, were identified through mass analysis (LC-ESI-MS/MS) and such analyzed results are shown in Tables 2 and 3 below.

**[TABLE 2] Significantly increased proteins in lung cancer identified by LC-ESI-MS/MS**

| **Identified Proteins** | **IPI Number** | **Peptide hits HEC** | **Peptide hits SQLC** | **Coverage (%)** | **Cancer fold** |
|---|---|---|---|---|---|
| Gene_Symbol=C7 Complement component C7 | IPI00296608 | 0 | 19 | 12 | - |
| | | | | 11 | |
| Gene_Symbol=ITIH3 Isoform 1 of Inter-alpha-trypsin inhibitor heavy chain H3 | IPI00028413 | 3 | 31 | 13 | 10 |
| | | | | 11 | |
| Gene_Symbol=C9 Complement component C9 | IPI00022395 | 4 | 27 | 15 | 6.8 |
| | | | | 11 | |
| Gene_Symbol=LRG1 Leucine-rich alpha-2-glycoprotein | IPI00022417 | 4 | 15 | 9.5 | 4 |
| | | | | 31 | |
| Gene_Symbol=SERPINA7 Thyroxine-binding globulin | IPI00292946 | 3 | 11 | 15 | 3.7 |
| | | | | 18 | |
| Gene-Symbol=IGHA2 Uncharacterized protein IGHA2 (Fragment) | IPI00829711 | 16 | 48 | 9.7 | 3 |
| | | | | 9.7 | |
| Gene_Symbol=SERPINA3 cDNA FLJ35730 fis, clone TESTI2003131, highly similar to ALPHA-1-NTICHYMOTRYPSIN | IPI00550991 | 184 | 335 | 46 | 2.0 |
| | | | | 52 | |
| Gere_Symbol=HPR 47 kDa protein | IPI00641737 | 288 | 513 | 45 | 1.8 |
| | | | | 65 | |
| Gene_Symbol=CP Ceruloplasmin | IPI00017601 | 168 | 260 | 49 | 1.5 |
| | | | | 54 | |

**[TABLE 3] Significantly decreased proteins in lung cancer identified by LC-ESI-MS/MS**

| **Identified Proteins** | **IPI Number** | **Peptide Hits HEC** | **Peptide Hits SQLC** | **Coverage (%)** | **Healthy fold** |
|---|---|---|---|---|---|
| Gene_Symbol=TTN Isoform 2 of Titin | IPI00023283 | 8 | 0 | 0.13 | - |
| | | | | 0.02 | |
| Gene_Symbol=GPLD1 Isoform 1 of Phosphatidylinositol-glycan-specific phospholipase D | PI00299503 | 6 | 0 | 3.1 | - |
| | | | | 5.2 | |
| Gene_Symbol=SERPINA4 Kallistatin | IPI00328609 | 13 | 0 | 17 | - |
| | | | | 12 | |
| Gene_Symbol=IGFALS cDNA FLI53838, highly similar to Insulin-like growth factor-binding proteincomplex acid labile chain | IPI00020996 | 13 | 2 | 9.5 | 6.5 |
| | | | | 5 | |
| Gene_Symbol=APOL1 Isoform 2 of Apolipoprotein-L1 | IPI00186903 | 28 | 8 | 25 | 3.5 |
| | | | | 16 | |
| Gene_Symbol=SERPINF2 SERPINF2 protein | IPI00029863 | 36 | 15 | 27 | 2.4 |
| | | | | 36 | |
| Gene_Symbol=AFM Afamin | IPI00019943 | 64 | 29 | 26 | 2 |
| | | | | 21 | |
| Gene_Symbol=APOB Apolipoprotein B-100 | IPI00022229 | 606 | 303 | 31 | 2 |
| | | | | 27 | |
| Gene_Symbol=SERPINF1 Pigment epithelium-derived factor | IPI00006114 | 17 | 8 | 8.4 | 2 |
| | | | | 8.9 | |
| Gene_Symbol= AHSG Alpha-2-HS-glycoprotein | IPI00022431 | 35 | 19 | 13 | 1.8 |
| | | | | 13 | |
| Gene_Symbol=CLU Clusterin | IPI00291262 | 48 | 27 | 15 | 1.7 |
| | | | | 21 | |

### Example 3: Investigation of target protein in lung cancer cell-line

The protein marker according to the present invention was investigated using a normal lung epithelium cell-line (L132 cell), a liver cancer cell-line (HepG2 cell), and five types of lung cancer cell-lines including bronchoalveolar carcinoma cell-lines (A549 cell and H358 cell) (i.e., A549, H358, H157, H1229 and H460), respectively. More particularly, after recovering mRNAs from different cell-lines, expression of GPLD1 protein was identified through real-time PCR.

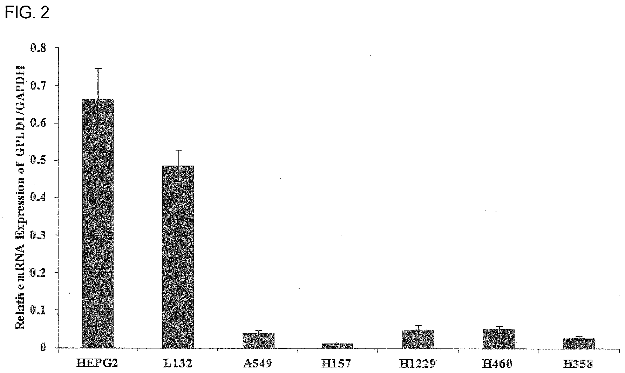

Experimental results are shown in FIG. 2 and it was demonstrated that, since plenty of GPLD1 is generated in the liver, this protein is expressed higher in a liver cell (HEPG2) and normal cell-line (L132) than the lung cancer cell-lines.

### Example 4: Investigation of target protein in human serum

In order to identify the analyzed results by a mass analyzer, expression patterns of GPLD1 in both serums obtained from a normal person and an SQLC patient were investigated through Western blot assay and using an ELISA method.

### (4-1) Results of Western blot assay

After weighing 50 µg of blood protein obtained from each of 56 normal persons and 56 lung cancer patients, respectively, the protein was separated in SDS gel through electrophoresis. The protein in the gel after completing the electrophoresis was shifted to a nitrocellulose membrane (Whatman, Germany), followed by a reaction thereof with a diluted antibody (1:500) (Abcam, USA) at 4°C for 16 hours. Next, after reacting the resultant material with an anti-mouse IgG-HRP linked antibody (Cellsignalling, USA) at room temperature for 1 hour, the obtained product was identified by an ECL or ECL plus Western blot assay system (Amersham Biosciences, UK).

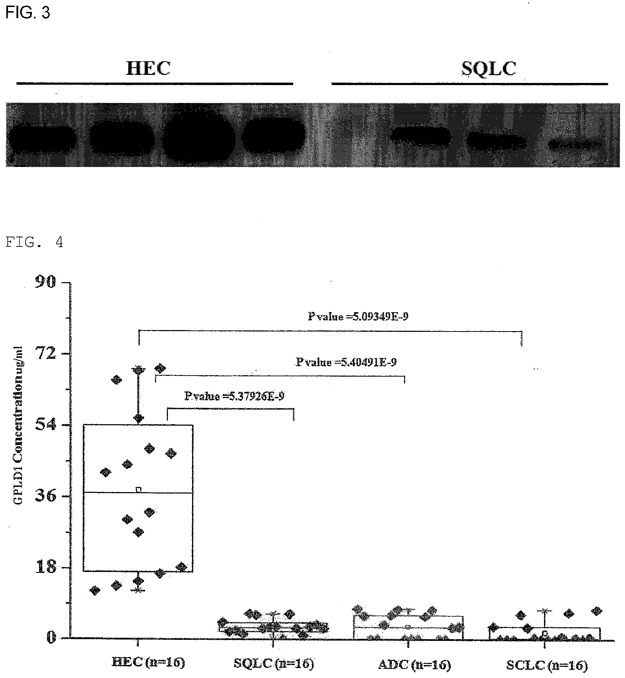

Results of the Western blot assay are shown in FIG. 3. As shown in FIG. 3, it was observed that the serum of the SQLC patients exhibited lower expression of GPLD1, as compared to those of the normal persons.

### (4-2) Results of ELISA assay

Using a sandwich ELISA kit commercially available in the market (USCN Life science Inc., USA), GPLD1 in the serum was measured. According to instructions stated in a manual provided in the present kit, an experimental method was executed. Briefly, 1000-fold diluted blood was placed in a prepared plate, followed by a reaction thereof at room temperature for 2 hours. After removing the sample and washing out the plate, 100 µl of a biotin antibody working solution was added thereto, followed by a reaction thereof at room temperature for 1 hour. Again, after removing the sample and washing out the plate, 100 µl of an HRP-avidin working solution was added thereto, followed by a reaction thereof at room temperature for 1 hour. Staining the cleaned plate was performed by adding a TMB substrate and the staining was stopped by adding 50 µl of stop solution thereto. Then, an optical density at 450 nm was read and results thereof were assessed.

The assessed results are shown in FIG. 4, and, as a result of identifying 16 GPLD1 expression patterns in serums obtained from each of the normal person (HEC), SQLC patient, lung adenocarcinoma (ADC) patient and SCLC patient, it was demonstrated that protein expression in the serums obtained from lung cancer groups (SQLC, ADC, and SCLC) was remarkably reduced, as compared to the normal person.

### Example 5: Investigation of target protein in lung cancer tissue

In order to identify as to whether the expression pattern of GPLD1 protein qualified in the lung cancer cell-line is also exhibited in a lung cancer patient, protein expression levels in lung cancer tissues and adjacent normal tissues obtained from total 22 lung cancer patients were identified through Western blot assay.

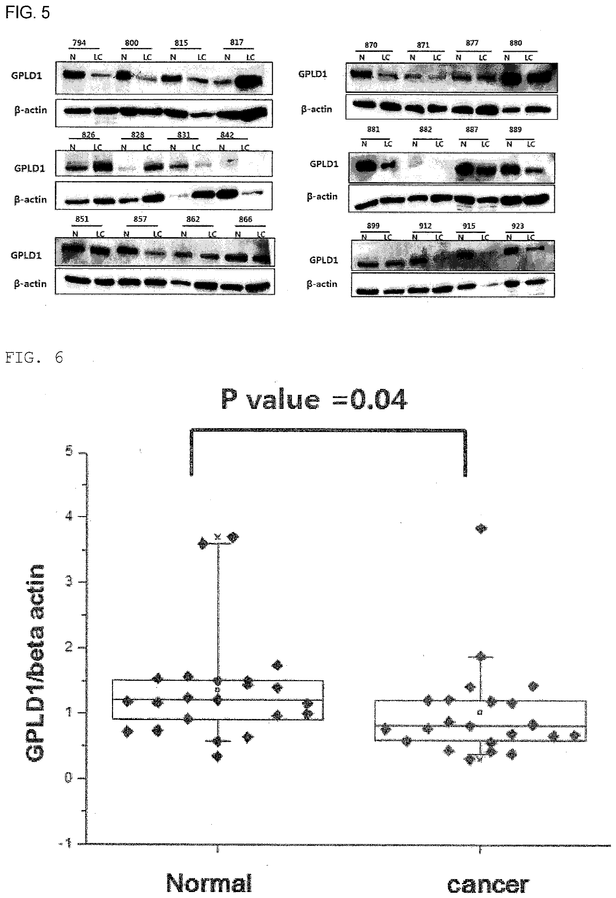

First, protein was removed from the lung cancer tissues and adjacent normal tissues obtained from 22 lung cancer patients. Briefly, after washing the lung cancer tissues with PBS to remove a blood portion thereof, homogenization was performed in a RIPA buffer containing protease inhibitor added thereto. Thereafter, for each sample, a quantification method through a Bradford assay was applied to identify expression of each of GPLD1 and beta actin (reference gene) in normal tissue and lung cancer tissue, respectively, on the same amount of protein, and therefore, expression patterns of GPLD1 have been compared to each other with regard to the same amount of protein. Results of Western blot assay are shown in FIG. 5.

Further, Western blot assay results of GPLD1 were subjected to quantification using a densitometry program and, more particularly, the specific gravity of GPLD1 in each blot was complemented with the specific gravity of beta actin as a reference gene and results thereof are shown in FIG. 6.

As shown in FIGS. 5 and 6, it was identified that lung cancer tissue obtained from a lung cancer patient has exhibited lower expression of GPLD1 protein than adjacent normal tissues.

### Example 6: Investigation of target protein in a lung cancer mouse model

In order to establish a mechanism for an expression pattern of GPLD1 in a human, investigation was performed using a lung cancer-derived mouse.

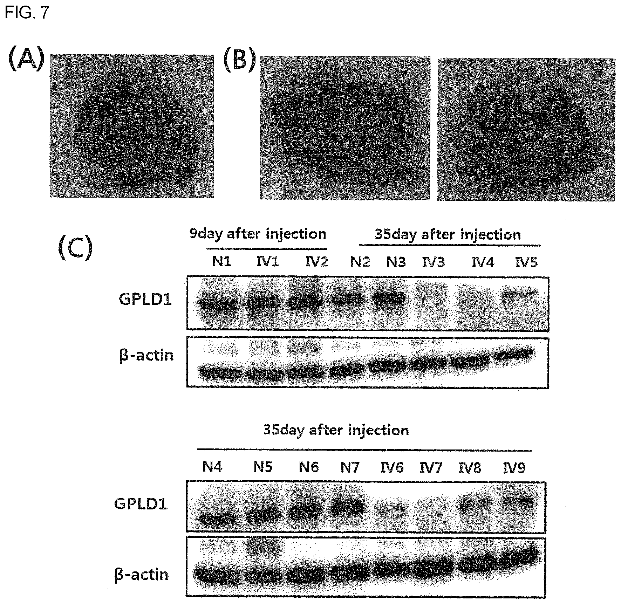

More particularly, lung cancer was caused by IV injecting lung cancer cell-line (LLC) into a tail vein of the mouse. At day 9 or day 35 after the injection, the mouse was sacrificed and an occurrence of cancer in the lung was checked. FIG. 7 shows (A) the lung of a normal mouse and (B) the lung of a lung cancer-derived mouse.

As such, the lung of the lung cancer-derived mouse was excised at the day 9 or 35 after IV injecting the lung cancer cell-line, followed by observation of an expression pattern of GPLD1 through Western blot. Results of the observation are shown in FIG. 7(C).

As shown in FIG. 7(C), it was demonstrated that a mouse also exhibited a remarkable decrease in an expression of GPLD1 after inducing the lung cancer in the mouse, as compared to a normal group.

## Claims

1. Use of a composition comprising an antibody specifically able to bind to glycosylphosphatidylinositol (GPI) specific phospholipase D1 (GPLD1) protein to detect a decreased expression of GPLD1 in a blood serum sample or cancer tissue sample for the early diagnosis of squalus epithelium cell lung cancer.

2. Use of a kit comprising an antibody specifically able to bind to GPLD1 protein to detect a decreased expression of GPLD1 in a blood serum sample or cancer tissue sample for the early diagnosis of squalus epithelium cell lung cancer.

3. Use of a micro-array comprising an antibody specifically able to bind to GPLD1 protein to detect a decreased expression of GPLD1 in a blood serum sample or cancer tissue sample for the early diagnosis of squalus epithelium cell lung cancer.

4. Use of a protein chip comprising an antibody specifically able to bind to GPLD1 protein to detect a decreased expression of GPLD1 in a blood serum sample or cancer tissue sample for the early diagnosis of squalus epithelium cell lung cancer.

5. Use of a composition comprising a primer or probe specific to a nucleic acid encoding GPLD1 protein to detect a decreased expression of GPLD1 in a blood serum sample or cancer tissue sample for the early diagnosis of squalus epithelium cell lung cancer.

6. Use of a kit comprising a primer or probe specific to a nucleic acid encoding GPLD1 protein to detect a decreased expression of GPLD1 in a blood serum sample cancer tissue sample for the early diagnosis of squalus epithelium cell lung cancer.

7. Use of a micro-array comprising a primer or probe specific to a nucleic acid encoding GPLD1 protein to detect a decreased expression of GPLD1 in a blood serum sample or cancer tissue sample for the early diagnosis of squalus epithelium cell lung cancer.

8. Use of a DNA chip comprising a primer or probe specific to a nucleic acid encoding GPLD1 protein to detect a decreased expression of GPLD1 in a blood serum sample or cancer tissue sample for the early diagnosis of squalls epithelium cell lung cancer.

## Patentansprüche

1. Verwendung einer Zusammensetzung umfassend einen Antikörper, welcher insbesondere fähig ist, Glycosylphosphatidylinositol (GPI) spezifisch an spezifische Phospholipase D1 (GPLD1) zu binden, um eine verminderte Expression von GPLD1 in einer Blutserumsprobe oder Krebsgewebeprobe fürdie Frühdiagionse von Squalus epithelium-Zellen Lungenkrebs zu erkennen.

2. Verwendung eines Kits, welches einen Antikörper aufweist welcher insbesondere fähig ist, spezifisch an GPLD1 zu binden, um eine verminderte Expression von GPLD1 in einer Blutserumsprobe oder Krebsgewebeprobe für die Frühdiagionse von Squalus epithelium-Zellen Lungenkrebs zu erkennen.

3. Verwendung einer Mikroansammlung, welches einen Antikörper aufweist, welcher insbesondere fähig ist, an GPLD1 zu binden, um eine verminderte Expression von GPLD1 in einer Blutserumsprobe oder Krebsgewebeprobe für die Frühdiagionse von Squalus epithelium-Zellen Lungenkrebs zu erkennen.

4. Verwendung eines Proteinchips, welches einen Antikörper aufweist, welcher insbesondere fähig ist, an GPLD1 zu binden, um eine verminderte Expression von GPLD1 in einer Blutserumsprobe oder Krebsgewebeprobe für die Frühdiagionse von Squalus epithelium-Zellen Lungenkrebs zu erkennen.

5. Verwendung einer Zusammensetzung, welche einen Primer oder eine spezifische Probe einer GPLD1 Protein kodierende Nukleinsäure umfasst, um eine verminderte Expression von GPLD1 in einer Blutserumsprobe oder Krebsgewebeprobe für
die Frühdiagionse von Squalus epithelium-Zellen Lungenkrebs zu erkennen.

6. Verwendung eines Kits, welches einen Primer oder eine Probe spezifisch für eine GPLD1 Protein kodierende Nukleinsäure umfasst, um eine verminderte Expression von GPLD1 in einer Blutserumsprobe oder Krebsgewebeprobe für
die Frühdiagionse von Squalus epithelium-Zellen Lungenkrebs zu erkennen.

7. Verwendung einer Mikro-Matrix, welche einen Primer oder eine Probe spezifisch für eine GPLD1 Protein kodierende Nukleinsäure umfasst, um eine verminderte Expression von GPLD1 in einer Blutserumsprobe oder Krebsgewebeprobe für
die Frühdiagionse von Squalus epithelium-Zellen Lungenkrebs zu erkennen.

8. Verwendung eines DMA-Chips, welche einen Primer oder eine Probe spezifisch für eine GPLD1 Protein kodierende Nukleinsäure umfasst, um eine verminderte Expression von GPLD1 in einer Blutserumsprobe oder Krebsgewebeprobe für
die Frühdiagionse von Squalus epithelium-Zellen Lungenkrebs zu erkennen.

## Revendications

1. Utilisation d'une composition comprenant un anticorps spécifique capable pour attacher à glycosylphosphatidylinositol (GPI) la phospholipase spécifique D1 (GPLD1) protéine pour détecter une expression réduite de GPLD1 dans un échantillon de sérum sanguin ou un échantillon de tissu du cancer pour le diagnose précoce du cancer du poumon Squalus cellules épithéliales.

2. Utilisation d'un kit comprenant un anticorps spécifique capable pour attacher à GPLD1 protéine pour détecter une expression réduite de GPLD1 dans un échantillon de sérum sanguin ou un échantillon de tissu du cancer pour le diagnose précoce du cancer du poumon Squalus cellules épithéliales.

3. L'utilisation d'un micro-collection comprenant un anticorps spécifique capable pour attacher à GPLD1 protéine pour détecter une expression réduite de GPLD1 dans un échantillon de sérum sanguin ou un échantillon de tissu du cancer pour le diagnose précoce du cancer du poumon Squalus cellules épithéliales.

4. Utilisation d'une puce de protéine comprenant un anticorps spécifique capable pour attacher à GPLD1 protéine pour détecter une expression réduite de GPLD1 dans un échantillon de sérum sanguin ou un échantillon de tissu du cancer pour le diagnose précoce du cancer du poumon Squalus cellules épithéliales.

5. Utilisation d'une composition comprenant une amorce ou une sonde spécifique d'une protéine GPLD1 acide nucléique codant pour détecter une expression réduite de GPLD1 dans un échantillon de sérum sanguin ou un échantil-Ion de tissu du cancer pour le diagnose précoce du cancer du poumon Squalus cellules épithéliales.

6. Utilisation d'un kit comprenant une amorce ou une sonde spécifique d'une protéine GPLD1 acide nucléique codant pour détecter une expression réduite de GPLD1 dans un échantillon de sérum sanguin ou un échantillon de tissu du cancer pour le diagnose précoce du cancer du poumon Squalus cellules épithéliales.

7. L'utilisation d'un micro-collection 7. Utilisation d'une micro-collection comprenant une amorce ou une sonde spécifique d'une protéine GPLD1 acide nucléique codant pour détecter une expression réduite de GPLD1 dans un échantillon de sérum sanguin ou un échantillon de tissu du cancer pour le diagnose précoce du cancer du poumon Squalus cellules épithéliales.

8. Utilisation d'un puces ADN, qui comprend une amorce ou une sonde spécifique d'une protéine GPLD1 acide nucléique codant pour détecter une expression réduite de GPLD1 dans un échantillon de sérum sanguin ou un échantillon de tissu du cancer pour le diagnose précoce du cancer du poumon Squalus cellules épithéliales.
